# EUROPÄISCHE PATENTANMELDUNG

(11) **EP 1 000 586 A2**
(43) Veröffentlichungstag der Anmeldung: **17.05.2000**
(21) Anmeldenummer: 99122610.1
(22) Anmeldetag: 13.11.1999
(51) Int. Cl.: A61B 18/00

(54) **Absaugvorrichtgung für Chirurgiegeräte**

(30) Priorität: 16.11.1998 DE 19852706
(71) Anmelder: ATMOS MEDIZINTECHNIK GmbH & Co., 79853 Lenzkirch (DE)
(72) Erfinder: Möller, Mario, 79843 Löffingen (DE); Rheiner, Norbert, Dipl.-Ing.(BA), 79848 Bonndorf (DE); Beller, Jürgen, Dipl.-Ing.(FH), 72810 Gomaringen (DE); Kühner, Ralf, Dipl.-Ing.(FH), 70565 Stuttgart (DE)
(74) Vertreter: Patentanwälte Westphal, Mussgnug & Partner

(57) **Zusammenfassung**

Absaugvorrichtung zum Absaugen luftgetragener oder luftgelöster Abfallprodukte einer mit einem strombetriebenen Chirurgiegerät durchgeführten chirurgischen Behandlung, wobei die Absaugvorrichtung wenigstens eine nach Maßgabe eines Aktivierungssignals aktivierbare Absaugeinrichtung und eine Aktivierungseinheit zur Bereitstellung des Aktivierungssignals (A) aufweist, wobei die Aktivierungseinheit einen an einer Netzanschlußleitung des Chirurgiegeräts anbringbaren Sensor (3) zur Erfassung der Strom- und/oder Spannungsverläufe in der Netzanschlußleitung aufweist.

## Beschreibung

Die vorliegende Erfindung betrifft eine Absaugvorrichtung für Chirurgiegeräte gemäß den Merkmalen des Oberbegriffs des Anspruchs 1.

Derartige Vorrichtungen dienen dazu, die bei chirurgischen Prozessen mitunter entstehenden dispergen bzw. gas- oder aerosolförmigen Nebenprodukte abzusaugen. Besonders beim Einsatz von Laser- oder Elektrochirurgie-Geräten werden Pyrolyseprodukte freigesetzt, die neben der Kontaminationsgefahr mit gefährlichen Viren (z.B. mit HPV = Human Papilloma Virus) auch eine Sichtbehinderung im Operationsfeld sowie eine olfaktorische Belastung für das Operationspersonal darstellen. Darüber hinaus entstehen unerwünschte luftgetragene Reaktionsprodukte beim Mischen und Verarbeiten von zweikomponentigen Klebstoffen und Zementen, z.B. in der implantiven Chirurgie, und beim zerspanenden Bearbeiten von Knochenmaterial. Auch Reaktangase von Chirurgiegeräten und medizinisch eingesetzte verdampfende Desinfektions- und Reinigungsmittel können eine Arbeitsbehinderung und eine gesundheitliche Gefahr für das Operationspersonal darstellen.

Zur Absaugung derartiger unerwünschter luftgetragener Nebenprodukte chirurgischer Prozeduren werden üblicherweise Absaugsysteme (Plume-Evacuation-Units) eingesetzt, die über eine Vakuum-Pumpe sowie über einen Luftkanal und meistens einen Filter verfügen.

Im Hinblick auf störende Begleiterscheinungen derartiger Absaugvorrichtungen, wie Schallemission und einen möglicherweise störenden Luftzug, der zu Staubverwirbelungen und einem unsterilen Operationsfeld führen kann, ist es angestrebt, die Absaugvorrichtung bedarfsgerecht zu steuern, was bei bekannten Vorrichtungen mittels eines durch den Operateur oder dessen Assistenz zu betätigenden Hand- oder Fußschalter erfolgt.

Aus der Patentschrift US 5,160,334 ist eine kombinierte Vorrichtung aus Chirurgiegerät und Absaugvorrichtung beschrieben, wobei bei Betätigen eines Chirurguieinstruments des Chirurgiegeräts die Absaugvorrichtung eingeschaltet wird, die bis zum Ablauf einer vorgebbaren Zeitdauer nach Ausschalten des Chirurgieinstruments eingeschaltet bleibt. Der Nachteil derartiger kombinierter Geräte besteht darin, daß jedes Chirurgiegerät eine eigene Absaugvorrichtung aufweisen muß und daß ältere Laser- oder Elektro-Chirurgiegeräte, die über keine derartige Absaugvorrichtung verfügen, nur mit erheblichem Aufwand, oder gar nicht, nachrüstbar sind.

Bekannte separate Absaugvorrichtungen zur bedarfsgerechten Absaugung von luftgetragenen Nebenprodukten chirurgischer Operationen verwenden Hochfrequenz-Sensoren, die an einer Verbindungsleitung des Chirurgiegeräts zu dem jeweiligen Chirurgieinstrument, z.B. einem Laser-Skalpell, angeordnet sind, und die die elektromagnetische Emission dieser Verbindungsleitung bei Betätigen des Chirurgieinstruments registrieren, um eine Absaugvorrichtung einzuschalten, die die luftgetragenen Nebenprodukte absaugt. Derartige Vorrichtungen sind aus den Patentschriften US 5,620,441 und US 5,318,516 bekannt.

Des weiteren sind Absaugvorrichtungen bekannt, bei denen eine Lichtschranke (Patentschrift US 5,108,389) oder ein Rauchdetektor (Patentschrift US 5,613,966) vorgesehen sind, um Rauch an der Operationsstelle zu erkennen und davon abhängig die Absaugvorrichtung zu starten.

Die bekannten separaten Absaugvorrichtungen haben jedoch den Nachteil, daß zusätzliche Vorrichtungen, wie Hochfrequenzsensoren, Lichtschranken oder Rauchdetektoren und deren Verkabelungen im Bereich der Operationsstelle erforderlich sind.

Lichtschranken oder Rauchdetektoren müssen möglichst nahe an der Operationsstelle angeordnet werden und können dort hinderlich sein. Weiterhin beginnt die Absaugung erst, wenn sich bereits Rauch entwickelt hat und nicht schon mit Betätigung des Chirurgieinstruments. An der Verbindungsleitung angeordnete Hochfrequenzsensoren verschlechtern die Handhabbarkeit des von dem Operateur geführten Instruments.

Ziel der vorliegenden Erfindung ist es daher, eine Absaugvorrichtung zur Verfügung zu stellen, die separat für verschiedene Chirurgieinstrumente einsetzbar ist, die keine zusätzlichen Vorrichtungen und Verkabelungen im Bereich der Operationsstelle erfordert und die insbesondere die oben genannten Nachteile nicht aufweist.

Dieses Ziel wird durch eine Vorrichtung gemäß den Merkmalen des Anspruchs 1 gelöst.

Danach weist die Aktivierungseinheit einen an einer Netzanschlußleitung des Chirurgiegeräts anbringbaren Sensor zur Erfassung der Strom- und/oder Spannungsverläufe in der Netzanschlußleitung auf. Bei Betätigen eines Chirurgieinstruments, beispielsweise eines Laserskalpells, eines strombetriebenen Chirurgiegeräts, wird Energie in Form von Licht und/oder Wärme an den Patienten oder die Umwelt abgegeben, wobei bei ordnungsgemäßer Verwendung an einer Operationsstelle luftgetragene Nebenprodukte erzeugt werden, die es gilt abzusaugen. Die erforderliche Energie wird von dem Chirurgiegerät aus dem Spannungsnetz über die Netzanschlußleitung aufgenommen, was zu einer Erhöhung der Stromaufnahme über die Netzanschlußleitung führt. Diese erhöhte Stromaufnahme wird durch den an der Netzanschlußleitung angeordneten Sensor registriert, um ein Einschalten der Absaugvorrichtung zu bewirken. Das Aktivierungssignal für die Absaugvorrichtung wird in der Aktivierungseinheit abhängig von einem Sensorsignal, das von dem Strom- und/oder Spannungsverlauf abhängig ist, erzeugt. Registriert der Sensor nach einem Abschalten des Chirurgieinstruments ein Absinken der Stromaufnahme, wird die Absaugvorrichtung, vorzugsweise zeitverzögert, wieder abgeschaltet.

Die Anordnung eines Sensor an der Netzanschlußleitung, bzw. an einer beliebigen stromführenden Verbindung, die zur Stromaufnahme des Chirurgiegeräts aus dem Spannungsnetz dient, bringt keine Behinderung im Bereich des Operationsfelds mit sich, da die Netzanschlußleitung üblicherweise weit davon entfernt ist, und behindert auch nicht die Handhabung des Chirurgieinstruments, da das Chirurgieinstrument nicht unmittelbar an die Netzanschlußleitung angeschlossen ist.

Vorteilhafte Ausgestaltungen der Erfindung sind Gegenstand der Unteransprüche.

Gemäß einer Ausführungsform der Erfindung ist vorgesehen, daß der Sensor als Niederfrequenzsensor ausgebildet ist, der die Strom- und/oder Spannungsverläufe in der Netzanschlußleitung induktiv erfasst. Ein derartiger Sensor, der im einfachsten Fall aus einer die Netzanschlußleitung umgebenden Stromschleife mit zwei Anschlußklemmen besteht, besitzt den Vorteil, daß er leicht an der Netzanschlußleitung des Chirurgiegeräts anbringbar ist und daß zu seiner Verwendung kein Auftrennen der Netzanschlußleitung erforderlich ist.

Die Aktivierungseinheit weist vorzugsweise eine an den Sensor angeschlossene Auswerteeinheit auf, in der die durch den Sensor erfaßten Strom- und/oder Spannungsverläufe zur Bereitstellung des Aktivierungssignals ausgewertet werden.

Üblicherweise nehmen betriebsbereite Elektrochirurgiegeräte permanent Strom aus dem Spannungsnetz auf. Hierdurch wird eine sofortige Einsatzbereitschaft des oder der Chirurgieinstrumente eines Chirurgiegeräts gewährleistet. Außerdem können im betriebsbereiten Zustand des Chirurgiegeräts Voreinstellungen, beispielsweise hinsichtlich der Intensität, für einen späteren Einsatz des oder der Chirurgieinstrumente vorgenommen werden.

Bei Betätigen eines Chirurgieinstruments steigt der von dem Chirurgiegerät über die Netzanschlußleitung aufgenommene Strom bzw. steigt die über die Netzanschlußleitung aufgenommene Leistung an. Bei Abschalten des Chirurgieinstruments sinkt die Strom- bzw. Leistungsaufnahme wieder ab. Zudem kann es beim Einschalten des Chirurgieinstruments zu kurzzeitigen Stromspitzen (Stromimpulsen) kommen und es kann kurzzeitig zu einem starken Absinken der über der Netzanschlußleitung anliegenden Netzspannung kommen.

Je nach verwendeten Sensor werden eine oder mehrere der genannten Größen erfasst, wobei der Auswerteeinrichtung ein von der erfaßten Größe abhängiges Sensorsignal zugeführt wird. Bei Verwendung mehrerer Sensoren zur Erfassung verschiedener Größen, wie Stromstärke und Spannungsstärke, werden der Auswerteeinrichtung eine entsprechende Anzahl Sensorsignale zugeführt. Die Auswerteeinrichtung ist dazu ausgebildet ein oder mehrere von dem Sensor gelieferte Sensorsignale zur Erzeugung des Aktivierungssignals auszuwerten.

Die Auswerteeinrichtung weist wenigstens einen Speicher auf, in dem charakteristische Werte über die Strom- und/oder Leistungsaufnahme des Chirurgiegeräts abspeicherbar sind. Solche charakteristischen Werte sind z.B. die Strom- und/oder Leistungsaufnahme im Stand-By-Betrieb, also bei nicht aktiviertem Chirurgieanwendungsteil, und bei aktiviertem Chirurgieanwendungsteil. Die Auswerteeinheit ist ausgebildet, diese Werte bei der Erzeugung des Aktivierungssignals, beispielsweise bei der Festlegung der Schwelle bei welcher über das Aktivierungssignal die Absaugeinrichtung eingeschaltet oder auf ein hohes Niveau geschaltet wird, zu berücksichtigen.

Vorzugsweise weist die Auswerteeinheit mehrere Speicher auf, in denen die Werte verschiedener Chirurgiegeräte, für welche die Absaugvorrichtung eingesetzt werden soll, abspeicherbar sind. Die Speicherwerte können werkseitig vorprogrammiert sein oder durch die Auswerteeinrichtung "gescannt" werden. Dadurch wird eine vielseitige Anwendbarkeit der Absaugvorrichtung erreicht.

Beim Scannen erlernt die Auswerteeinrichtung die verschiedenen Signalpegel, die beim Betrieb des jeweiligen Chirurgiegeräts auftreten können. Sowohl der Start des Lernvorgangs als auch dessen Ablauf erfolgt je nach Ausführungsform der Auswerteeinrichtung manuell oder automatisch.

Die Auswerteeinheit der Absaugvorrichtung ist vorzugsweise dazu ausgebildet, automatisch zu erkennen an welches der Chirurgiegeräte, deren Werte gespeichert sind, sie angeschlossen ist. Bei einer anderen Ausführungsform weist sie Eingabemittel auf, um einzugeben, welches Chirurgiegerät angeschlossen ist.

Zur automatischen Erkennung des angeschlossenen Geräts werden beim Lernvorgang neben oberen und unteren Signalpegeln vorzugsweise Abtastwerte von Signalverläufen des Stroms der Netzanschlußleitung, beispielsweise der Signalverläufe unmittelbar nach dem Einschalten des Chirurgieeinstruments, abgespeichert. Die Stromverläufe der Netzstromaufnahme unmittelbar nach Einschalten eines Chirurgieinstruments sind charakteristisch für ein Chirurgiegerät und ermöglichen eine Unterscheidung gegenüber anderen Chirurgiegeräten. Die abgespeicherten Folgen können dabei vorprogrammiert sein oder erlernt werden.

Die Auswerteeinrichtung weist vorzugsweise eine Abtastvorrichtung zur Abtastung des Sensorsignals und einen temporären Speicher, beispielsweise einen FIFO-Speicher auf, in dem die innerhalb einer vorgebbaren Zeitdauer vor dem aktuellen Zeitpunkt erzeugten Abtastwerte speicherbar sind. Die Folge der Abtastwerte wird durch geeignete Vergleichsverfahren, beispielsweise ein Korrelationsverfahren, mit den Folgen der abgespeicherten Signalverläufe verglichen, um zu ermitteln, welches Chirurgiegerät angeschlossen ist. Der Vergleich kann dabei jedes mal dann erfolgen, wenn ein neuer Abtastwert in den temporären Speicher eingelesen wird und ein vorheriger aus dem temporären Speicher fällt.

Die vorliegende Erfindung wird nachfolgend in Ausführungsbeispielen anhand von Figuren näher erläutert. Es zeigen:
- Figur 1:: Blockdiagramm einer erfindungsgemäßen Absaugvorrichtung;
- Figur 2:: Blockdiagramm einer Ausführungsform einer Auswerteeinrichtung einer Aktivierungseinheit der Absaugvorrichtung;
- Figur 3:: Blockdiagramm einer Absaugvorrichtung gemäß einer weiteren Ausführungsform.

In den Figuren bezeichnen, sofern nicht anders angegeben gleiche Bezugszeichen gleiche Teile mit gleicher Bedeutung.

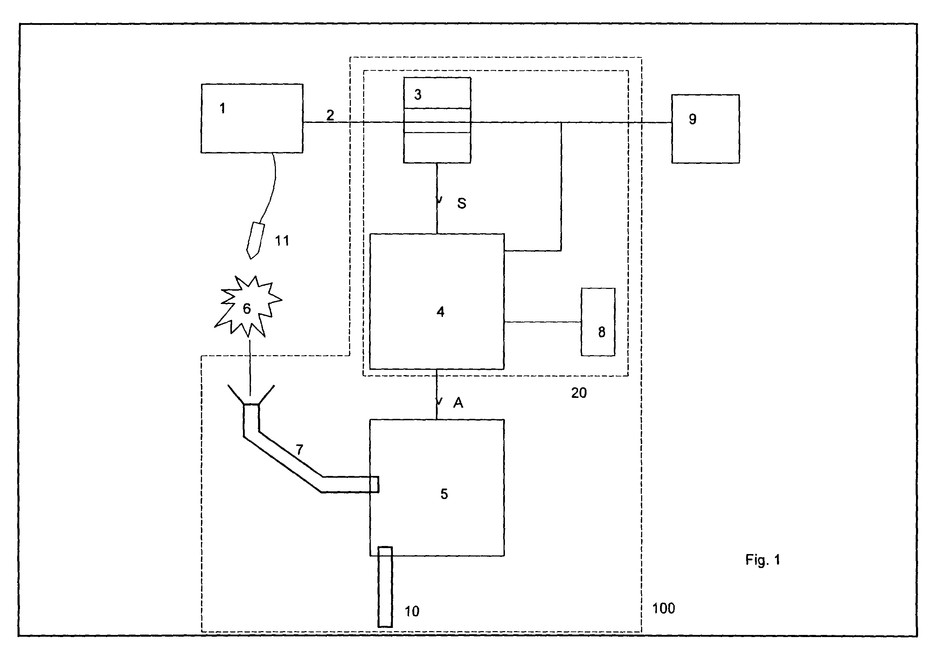

Figur 1 zeigt ein Blockdiagramm eines Ausführungsbeispiels einer erfindungsgemäßen Absaugvorrichtung 100 zum Absaugen luftgetragener oder luftgelöster Abfallprodukte einer chirurgischen Behandlung, die mit einen Chirurgieinstrument 11, wie einem Laserskalpell, eines strombetriebenen Chirurgiegeräts 1 durchgeführt wird. Das Chirurgiegerät 1 ist zur Stromversorgung über eine Netzanschlußleitung 2 an eine Netzspannungsversorgung 9 angeschlossen. Die Absaugvorrichtung 100 weist eine Absaugeinrichtung 5 mit einem Absaugkanal 7 zum Absaugen des Rauchs 6 oder der luftgetragenen Bestandteile von der Operationsstelle auf. Die Absaugeinrichtung 5 weist vorzugsweise Filter, zu Filterung der abgesaugten Luft auf, die über einen Abluftkanal 10 ins Freie oder weiteren Filtern zugeleitet wird.

Zur Ansteuerung der Absaugeinrichtung 5, insbesondere zur Ansteuerung eines Gebläses oder einer Turbine der Absaugeinrichtung 5 ist eine Aktivierungseinheit 20 vorgesehen, die einen an einer Netzanschlußleitung 2 des Chirurgiegeräts angeordneten Sensor 3 und eine an den Sensor 3 angeschlossene Auswerteeinrichtung 4 zur Auswertung eines Sensorsignals S aufweist. Die Ansteuerung der Absaugeinrichtung 5 erfolgt nach Maßgabe eines in der Auswerteeinheit 4 abhängig von dem Sensorsignal S erzeugten Aktivierungssignals A.

Der Sensor 3 ist vorzugsweise ein Niederfrequenz-Sensor zur induktiven Erfassung des Stromverlaufes in der Netzanschlußleitung 2. Beliebige andere Sensoren oder Meßgeräte zur Strom-, Spannungs- oder Leistungsmessung, wie 50-Hertz-Transformer oder Stromwandler sind ebenfalls einsetzbar.

Die Auswerteeinrichtung 4 ist dazu ausgebildet, das von der erfaßten Meßgröße abhängige Sensorsignal S zur Bereitstellung des Aktivierungssignals A auszuwerten. In betriebsbereitem Zustand des Chirurgiegerät 1, aber bei nicht betätigtem Chirurgieinstrument 11, nimmt das Chirurgieinstrument 1 einen Strom über die Netzanschlußleitung 2 auf, der zur Aufrechterhaltung des betriebsbereiten Zustands erforderlich ist. Bei anschließendem Betätigen des Chirurgieinstruments 11 wird Energie in Form von Wärme und/oder Licht an die Umgebung oder den Patienten abgegeben, wobei diese Energie von der Netzspannungsversorgung 9 über die Netzanschlußleitung aufgenommen wird, was zu einer Erhöhung der Stromaufnahme führt, die sich auf eine Änderung des Sensorsignals S auswirkt.

Die Auswerteeinheit 4 ist vorzugsweise eine programmierbare Schaltungsanordnung, die je nach Einsatzzweck der Absaugvorrichtung 100 und je nach verwendetem Chirurgieinstrument 11, verschieden ausgebildet oder verschieden einstell- oder programmierbar ist, um das Aktivierungssignal A aus dem Sensorsignal S zu erzeugen.

Eine erste Ausführungsform sieht vor, daß die Auswerteeinrichtung 4 zur Erzeugung des Aktivierungssignals A eine Schwellwertentscheidung durchführt, wobei ein zweiwertiges Aktivierungssignal A erzeugt wird, dessen Wert davon abhängt, ob das Sensorsignal S oberhalb oder unterhalb des vorgebbaren Schwellwerts liegt. Ein oberer Wert des Aktivierungssignals A, der auf eine erhöhte Strom- oder Leistungsaufnahme hindeutet, ist geeignet gewählt, die Absaugeinrichtung 5 einzuschalten. Ein unterer Wert des Aktivierungssignals A ist geeignet gewählt, die Absaugeinrichtung 5 abzuschalten.

Die beiden Werte des Aktivierungssignals A sind vorzugsweise so gewählt, daß die Absaugeinrichtung 5 bei einem der beiden Aktivierungssignalwerte auf eine erhöhte Absaugleistung und bei dem anderen der beiden Aktivierungssignalwerte auf eine verringerte Absaugleistung eingestellt wird. Hierdurch findet permanent eine Absaugung statt.

Gemäß einer weiteren Ausführungsform der Erfindung ist die Auswerteeinrichtung 4 derart ausgebildet, daß ein zu dem Sensorsignal S, und damit zu der Strom- oder Leistungsaufnahme, proportionales oder wenigstens annäherungsweise proportionales Aktivierungssignal A erzeugt wird. Eine Steuerung der Absaugeinrichtung 5 ist dabei derart ausgebildet, daß die Absaugleistung abhängig von dem Aktivierungssignal A einstellbar ist. Dem liegt zugrunde, daß bei gleicher Applikation die Rauchentwicklung an der Operationsstelle um so stärker ist, je mehr Strom bzw. Leistung über die Netzanschlußleitung für das Chirurgieinstrument 11 aufgenommen wird. Bei der genannten Ausführungsform ist die Absaugleistung an die Strom- oder Leistungsaufnahme und damit an die Rauchentwicklung angepaßt.

Die Auswerteeinrichtung 4 ist vorzugsweise dazu ausgebildet, einen Impuls des Sensorsignals S, der aus einem Stromimpuls auf der Netzanschlußleitung 2 beim Einschalten resultiert, zu detektieren und dann ein Aktivierungssignal A zum Einschalten oder Erhöhen der Absaugleistung der Absaugeinrichtung 5 zu erzeugen. Hierdurch ist ein schnelles "Einschalten" der Absaugeinrichtung 5 gewährleistet. Die Absaugleistung ist dann abhängig von der sich nach dem Impuls einstellenden Strom- oder Leistungsaufnahme über das Aktivierungssignal 5 einstellbar.

Das Aktivierungssignal A zur Ansteuerung der Absaugeinrichtung ist vorzugsweise so gewählt, daß die Absaugleistung ein unteres Niveau nicht unterschreitet, um so auch bei nicht betätigtem Chirurgieinstrument 11 einen Stand-By-Betrieb aufrecht zu halten. Eine Gebläseeinheit bzw. Turbine der Absaugeinrichtung 5 wird dabei bei etwa 20% ihrer maximalen Umdrehungszahl betrieben. Hierdurch wird ein stetiger Luftstrom erreicht, durch den Filterstufen der Absaugvorrichtung abtrocknen, wenn diese durch Herausfiltern feuchter Luftbestandteile durchnäßt sind. Weiterhin wird ausgehend von dem Stand-By-Betrieb im Bedarfsfall eine erhöhte oder die maximale Absaugleistung schneller erreicht.

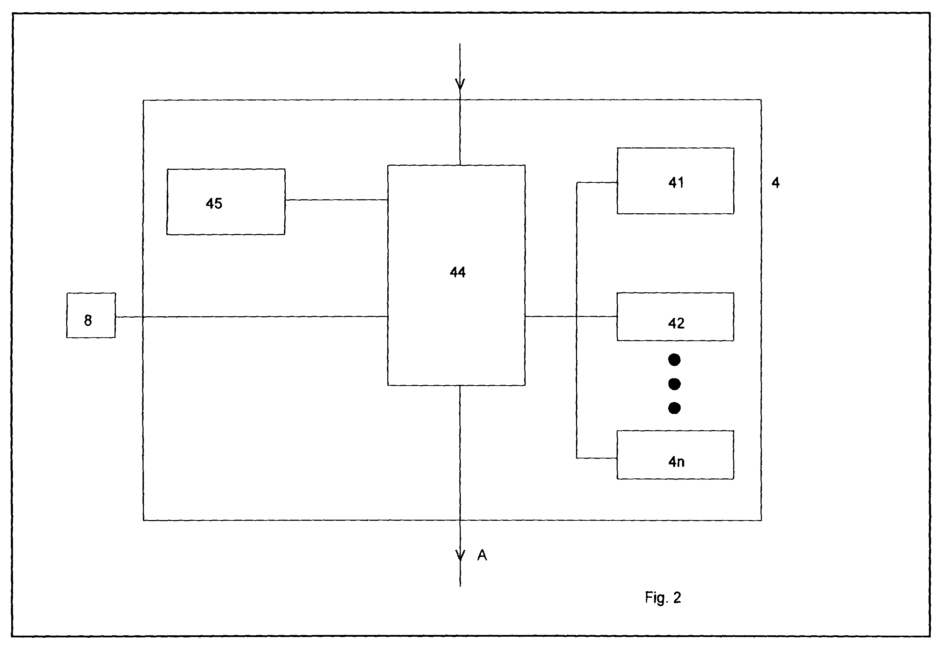

Figur 2 zeigt ein Ausführungsbeispiel einer Auswerteeinrichtung 4 der erfindungsgemäßen Absaugvorrichtung 100. Die Auswerteeinrichtung 4 weist Speicher 41, 42, ..., 4n auf, in denen charakteristische Werte für die Strom- und/oder Leistungsaufnahme von Chirurgiegeräten, abspeicherbar sind. Solche charakteristischen Werte sind z.B. die Strom- oder Leistungsaufnahme bei nicht aktiviertem und bei aktiviertem Chirurgieinstrument 11. Diese Werte werden bei der Erzeugung des Aktivierungssignals A, beispielsweise bei der Festlegung des Schwellwerts bei welcher über das Aktivierungssignal A die Absaugeinrichtung 5 eingeschaltet oder auf ein hohes Niveau geschaltet wird, herangezogen.

Die abgespeicherten Werte können werkseitig vorprogrammiert sein oder durch die Auswerteeinrichtung 4 "gescannt" werden.

Beim Scannen erlernt die Auswerteeinrichtung 4 die verschiedenen Signalpegel, die beim Betrieb des jeweiligen Chirurgiegeräts 1 auftreten können. Sowohl der Start des Lernvorgangs als auch dessen Ablauf erfolgt je nach Ausführungsform manuell oder automatisch. Die Auswerteeinrichtung 44 weist eine Verarbeitungseinheit 44 auf, in der ein Programm zur Durchführung des Lernverfahrens ablaufen kann.

Der Start des Lernvorgangs kann automatisch, beispielsweise beim Einschalten des Chirurgiegeräts, oder auch manuell durch Betätigen einer Taste 8 an der Auswerteeinrichtung 4 erfolgen.

Zum Erlernen der Signalpegel bzw. der charakteristischen Werte des Stromverlaufs ist vorgesehen, daß die Auswerteeinrichtung 4 die verschiedenen Signalpegel des Chirurgiegeräts bzw. die charakteristischen Werte programmgesteuert während des Betriebs des Chirurgiegeräts 1 erlernt und abspeichert. Wesentlich ist dabei die Erfassung und Speicherung des niedrigsten ermittelten Pegels, der den Pegel für den Stand-By-Betrieb des Chirurgiegeräts 1 darstellt und als Referenzwert für die Beurteilung dient, ob das Chirurgieinstrument betätigt wurde. Der höchste während des Lernverfahrens bei Betrieb des Chirurgiegeräts ermittelte und abgespeicherte Pegel, dient als Referenz für die Einstellung der maximalen Absaugleistung der Absaugeinrichtung über das Aktivierungssignal.

Gemäß einem weiteren Ausführungsform ist zum Erlernen der Signalpegel vorgesehen, den Lernvorgang durch Betätigen des Betätigungselements 8 zu starten, wobei anschließend das Chirurgiegerät 1 programmgesteuert seine verschiedenen Betriebszustände durchläuft und wobei dabei auftretende charakteristische Sensorsignale abgespeichert werden.

Die Auswerteeinrichtung 4 der Absaugvorrichtung 100 ist automatisch in der Lage zu erkennen, an welches der Chirurgiegeräte 1, deren Werte gespeichert sind, sie angeschlossen ist. Dazu sind neben oberen und unteren Signalpegeln der Chirurgiegeräte vorzugsweise Abtastwerte von Signalverläufen des Stroms in der Netzanschlußleitung, beispielsweise der Signalverläufe unmittelbar nach dem Einschalten des Chirurgieinstruments 11 des Chirurgiegeräts 1, abgespeichert. Die Stromverläufe der Netzstromaufnahme unmittelbar nach Einschalten eines Chirurgieinstruments 11 sind charakteristisch für ein Chirurgiegerät 1 und ermöglichen eine Unterscheidung gegenüber anderen Chirurgiegeräten 1. Die Folgen der Abtaswerte sind vorprogrammiert oder erlernt.

Die Auswerteeinrichtung 4 weist eine Abtastvorrichtung in der Verarbeitungseinheit 44 zur Abtastung des Sensorsignals S und einen temporären Speicher 45, beispielsweise einen FIFO-Speicher auf, in dem die während einer Zeitdauer vor dem aktuellen Zeitpunkt erzeugten Abtastwerte speicherbar sind. Die Folge der Abtastwerte wird durch geeignete Vergleichsverfahren, beispielsweise ein Korrelationsverfahren, mit den Folgen der in den Speichern 41, 42,..., 4n abgespeicherten Signalverläufe verglichen, um zu ermitteln, welches Chirurgiegerät 1 angeschlossen ist. Der Vergleich kann dabei jedesmal dann erfolgen, wenn ein neuer Abtastwert in den temporären Speicher eingelesen wird und ein vorheriger Wert herausfällt.

Bei einer weiteren nicht näher dargestellten Ausführungsform sind Tasten vorgesehen, um einzugeben, welches der Chirurgiegeräte, deren charakteristischen Werte in den Speichern abgespeichert sind, angeschlossen ist.

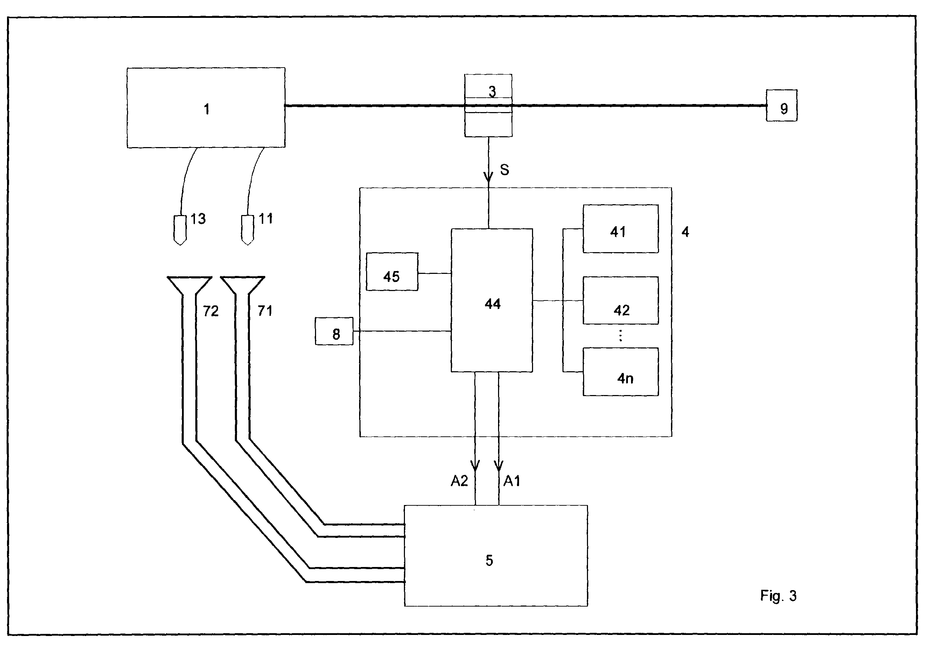

Figur 3 zeigt eine weitere Ausführungsform einer erfindungsgemäßen Absaugvorrichtung. Die Absaugvorrichtung dient zur Absaugung von luftgetragenen Bestandteilen, die beim Einsatz von Chirurgieinstrumenten 11, 13 eines Chirurgiegeräts 1 entstehen. Die Absaugvorrichtung 5 weist eine Absaugeinrichtung 5 mit zwei Absaugkanälen 71, 72 auf, wobei die Absaugung über die beiden Kanäle nach Maßgabe von Aktivierungssignalen A1, A2 unabhängig voneinander steuerbar ist. Vorzugsweise ist ein erster Absaugkanal 71 einem ersten Chirurgieinstrument 11 und ein zweiter Absaugkanal 72 einem zweiten Chirurgieinstrument 13 zur Absaugung zugeordnet. Die Chirurgieinstrumente 11, 13 weisen verschiedene charakteristische Signalverläufe für Strom und/oder Spannung bei Ein- oder Ausschalten auf, wobei jeweils Folgen von Abtastwerten dieser Signalverläufe für jedes der Chirurgieinstrumente in einem Speicher 41, 42,..., 4n abgespeichert sind. Die Werte können dabei vorprogrammiert sein oder, wie oben beschrieben, erlernt werden.

Der momentane Signalverlauf, dessen jeweils letzten Abtastwerte in dem temporären Speicher gespeichert sind, wird in regelmäßigen Zeitabständen mit den gespeicherten Folgen der charakteristischen Signalverläufe der Chirurgieinstrumente 11, 13 verglichen. Wird durch Vergleich des momentanen Signalverlaufs mit den charakteristischen Signalverläufen die Aktivierung oder Deaktivierung eines Chirurgieinstruments 11, 13 erkannt, wird der zugeordnete Absaugkanal 71; 72 dementsprechend aktiviert oder deaktiviert. Ist bereits ein Chirurgieinstrument 11; 13 aktiviert und hat sich nach dem Aktivieren, die Strom- oder Leistungsaufnahme auf einen bestimmten Wert eingeregelt, so überlagert sich beim nachfolgenden Einschalten eines weiteren Chirurgieinstruments 13; 11 dessen charakteristischer Signalverlauf dem weitgehend konstanten Signalverlauf des bereits eingeschalteten Chirurgieinstruments 11; 13. Geeignete, in der Verarbeitungseinheit implementierte Vergleichsalgorithmen, beispielsweise die Bildung einer Kreuzkorrelation zwischen der Abtastfolge in dem temporären Speicher und den in den Speichern 42, 42,..., 4n abgelegten Folgen, ermöglichen das Einschalten des weiteren Chirurgieinstruments 13; 11 auch dann zu erkennen, wenn bereits ein Chirurgieinstrument eingeschaltet ist.

Die in Figur 3 beschriebene Absaugvorrichtung ist auch einsetzbar, wenn mehrere Chirurgiegeräte 1 eingesetzt sind, die an eine gemeinsame Netzanschlußleitung angeschlossen sind, wobei charakteristische Werte der einzelnen Chirurgiegeräte 1 in den Speichern 41, 42,..., 4n abgelegt sind, um bei Betätigen des Chirurgieinstruments eines der Chirurgiegeräte zu erkennen, welches Chirurgieinstrument betätigt wurde und davon abhängig ein Aktivierungssignal A1; A2 zur Ansteuerung des zugeordneten Absaugkanals zu erzeugen.

## Patentansprüche

1. Absaugvorrichtung zum Absaugen luftgetragener oder luftgelöster Abfallprodukte einer mit einem strombetriebenen Chirurgiegerät (1) durchgeführten chirurgischen Behandlung, wobei die Absaugvorrichtung (100) wenigstens eine nach Maßgabe eines Aktivierungssignals (A; A1, A2) aktivierbare Absaugeinrichtung (5) und eine Aktivierungseinheit (20) zur Bereitstellung des Aktivierungssignals (A) aufweist,
**dadurch gekennzeichnet,** daß
die Aktivierungseinheit (20) einen an einer Netzanschlußleitung (2) des Chirurgiegeräts anbringbaren Sensor (3) zur Erfassung der Strom- und/oder Spannungsverläufe in der Netzanschlußleitung (2) aufweist.

2. Absaugvorrichtung nach Anspruch 1,
**dadurch gekennzeichnet,** daß
der Sensor (3) als Niederfrequenzsensor zur induktiven Erfassung der Strom- und/oder Spannungsverläufe ausgebildet ist.

3. Absaugvorrichtung nach einem der vorangehenden Ansprüche,
**dadurch gekennzeichnet,** daß
die Aktivierungseinheit (20) eine an den Sensor (3) angeschlossene Auswerteeinrichtung (4) zur Bereitstellung des Aktivierungssignals (A; A1, A2) aufweist.

4. Absaugvorrichtung nach einem der vorangehenden Ansprüche,
**dadurch gekennzeichnet,** daß
die Absaugeinrichtung (5) nach Maßgabe des Aktivierungssignals (A; A1, A2) ein- und ausschaltbar ist.

5. Absaugvorrichtung nach einem der vorangehenden Ansprüche,
**dadurch gekennzeichnet,** daß
eine Absaugleistung der Absaugeinrichtung (5) nach Maßgabe des Aktivierungssignals (A) auf ein hohes Niveau oder ein niedriges Niveau einstellbar ist.

6. Absaugvorrichtung nach einem der vorangehenden Ansprüche,
**dadurch gekennzeichnet,** daß
eine Absaugleistung der Absaugeinrichtung (5) nach Maßgabe des Aktivierungssignals (A) innerhalb vorgebbarer Grenzen wenigstens annäherungsweise stufenlos regelbar ist.

7. Absaugeinrichtung nach einem der vorangehenden Ansprüche,
**dadurch gekennzeichnet,** daß
die Absaugeinrichtung (5) wenigstens zwei Absaugkanäle (71, 72) aufweist, die nach Maßgabe von Aktivierungssignalen (A1, A2) unabhängig voneinander ansteuerbar sind.

8. Absaugvorrichtung nach einem der vorangehenden Ansprüche,
**dadurch gekennzeichnet,** daß
die Auswerteeinheit (4) wenigstens einen Speicher (41, 42,..., 4n) aufweist, in dem charakteristische Werte und/oder Folgen charakteristischer Werte für Strom- und/oder Spannungsverläufe verschiedener Chirurgiegeräte (1) und/oder verschiedener Chirurgieinstrumente (11, 13) eines Chirurgiegeräts (1) abspeicherbar sind.

9. Absaugvorrichtung nach einem der vorangehenden Ansprüche,
**dadurch gekennzeichnet,** daß
die Auswerteeinheit (4) eine programmierbare Steuerschaltung ist.

10. Absaugvorrichtung nach einem der vorangehenden Ansprüche,
**dadurch gekennzeichnet,** daß
die Auswerteeinheit (4) dazu ausgebildet ist, momentane mittels des Sensors (3) erfaßte Strom- und/oder Spannungswerte der Netzanschlußleitung (9) mit in dem wenigstens einen Speicher (41, 42,...,4n) abgespeicherten Strom- und/oder Spannungswerten zu vergleichen und nach Maßgabe des Vergleichs das Aktivierungssignal (A) zu erzeugen.

11. Absaugvorrichtung nach einem der vorangehenden Ansprüche,
**dadurch gekennzeichnet,** daß
die Auswerteeinheit (4) eine Verarbeitungseinheit (44) mit einer Abtasteinrichtung und einen temporären Speicher (45) zum Abspeichern einer Folge abgetasteter Sensorsignale aufweist und daß die Auswerteeinheit (4) ausgebildet ist, die Folge der in dem temporären Speicher abgespeicherten Abtastwerte mit der in dem wenigstens einen Speicher (41, 42,..., 4n) abgespeicherten Folge zu vergleichen und ein Aktivierungssignal (A; A1, A2) nach Maßgabe des Vergleichsergebnisses zu erzeugen.

12. Absaugeinrichtung nach einem der vorangehenden Ansprüche,
**dadurch gekennzeichnet,** daß
die Aktivierungseinheit ein Betätigungselement (8) aufweist, wobei nach Maßgabe einer Betätigung des Betätigungselements (8) Werte der Strom- und/oder Spannungsverläufe der Netzanschlußleitung (2) in dem Speicher (41, 42, ..., 4n) abspeicherbar sind.
